Europäisches Patentamt

European Patent Office

Office européen des brevets

(11) Publication number: **0 231 387**
**B1**

(12) **EUROPEAN PATENT SPECIFICATION**

(45) Date of publication of patent specification: **05.12.90**

(51) Int. Cl.⁵: **A 01 G 7/00, A 01 G 31/00**

(21) Application number: **86904391.9**

(22) Date of filing: **18.07.86**

(86) International application number:
**PCT/JP86/00377**

(87) International publication number:
**WO 87/00394 29.01.87 Gazette 87/03**

(54) **CULTURE MEDIUM SUPPORTS FOR PLANT TISSUE CULTURE.**

(30) Priority: **19.07.85 JP 158410/85**
**15.10.85 JP 227763/85**

(43) Date of publication of application:
**12.08.87 Bulletin 87/33**

(45) Publication of the grant of the patent:
**05.12.90 Bulletin 90/49**

(84) Designated Contracting States:
**DE FR GB NL SE**

(56) References cited:
**EP-A-0 117 766**
**DE-A-2 843 905**
**DE-A-3 207 623**

(73) Proprietor: **NIPPON STEEL CHEMICAL CO. LTD.**
**13-16 Ginza 5-chome Chuo-ku**
**Tokyo 104 (JP)**

(72) Inventor: **OISHI, Toru**
**2-1-218, Asashigaoka 1-chome**
**Asaka-shi Saitama-ken 351 (JP)**

(74) Representative: **Strehl, Schübel-Hopf, Groening**
**Maximilianstrasse 54 Postfach 22 14 55**
**D-8000 München 22 (DE)**

Courier Press, Leamington Spa, England.

**Description**

Technical field

This invention relates to a culture medium support used in combination with a liquid culture medium for plant tissue culture, more particularly to a culture medium support made of ceramic fibers, and further relates to a culture medium support for plant tissue culture usable also as an acclimatization culture medium for plantlets.

Background art

Agar has been used most commonly as a culture medium support in plant tissue culture and a combined use of liquid culture media and special supports such as glass fibers and absorbent cotton has also been known.

Agar, however, contains unidentified substances which hinder the propagation, regeneration, and/or growth of the root of plant tissue cells and even purified agar considerably retards the propagation, regeneration, and/or root formation in some plants. Besides, a culture medium containing agar is a gel and the waste matters and the harmful substances produced by the cultured tissues may diffuse so slowly as to make culture extremely difficult for some plants.

Moreover, in the plantlets obtained by tissue culture in a gelled culture medium such as agar, the roots regenerate from the cultured tissues and grow straight with no roothair in small numbers or they are the so-called underwater roots. These plantlets show extremely poor survival when directly transplanted to an acclimatization culture medium and acclimatized. Any culture medium adhering to the roots, if brought into the acclimatization culture medium, may cause decay and death of the plant as a result of contamination by fungi. A better removal of the culture medium from the plantlets will damage the plant more and result in retarded growth of the plant after acclimatization. Hence, with the conventional culture method using agar or the like, there was a need to use the acclimatization culture medium prepared from specially sterilized perlite or vermiculite at the time of acclimatization.

EP—A—117 766 discloses a culture substrate for plant tissue and the resulting plantlets which consists of synthetic fibers.

Glass fibers, when used as culture medium support present a problem in that the alkaline components pass into the medium while in use and change the pH and chemical composition of the culture medium. Absorbent cotton is known to yield worse results than agar culture media.

Therefore, an object of this invention is to provide a novel culture medium support to be used in combination with a liquid culture medium for plant tissue culture.

Another object of this invention is to provide a culture medium support which promotes the propagation, regeneration and root formation of plant tissues and improves the rate of survival during acclimatization and growth after acclimatization of plantlets.

A further object of this invention is to provide a culture medium support which enables one to carry out culture of plant tissues that can be cultured with difficulty by a gel culture medium such as agar.

A still further object of this invention is to provide a culture medium support which can be used continuously from the culture of tissues to the planting of the plant after acclimatization, saves labor in the acclimatization work, and improves the rate of survival at the time of acclimatization and the production rate after acclimatization.

Disclosure of invention

Thus, this invention provides a culture medium support to be used in combination with a liquid culture medium for plant tissue culture prepared from ceramic fibers which do not substantially release components harmful to plant tissue culture, wherein the ceramic fibers are silica-alumina fibers. These ceramic fibers do not release components harmful to the propagation, regeneration, and root formation of plant tissues in any step of tissue culture, for example, in the impregnation with a liquid culture medium and the subsequent sterilization. Moreover, it is important that the said ceramic fibers do not substantially react with the liquid culture medium to be used in combination and do not affect the pH of the liquid culture medium. In particular, aluminum ions dissolving out of the support will react with phosphate ions in the liquid culture medium to form water-insoluble aluminum phosphate, resulting in shortage of phosphate ions. Furthermore, release of alkali ions will change the pH of the liquid culture medium in use and hinder the propagation, regeneration, and root formation of plant tissues.

The ceramic fibers to be used in accordance with this invention are fibers based on silica and alumina such as silica-alumina ($SiO_2$—$Al_2O_3$) fibers, silica-alumina-zirconia ($SiO_2$—$Al_2O_3$—$ZrO_2$) fibers, and silica-alumina-chromia ($SiO_2$—$Al_2O_3$—$Cr_2O_3$) fibers. Silica-alumina fibers are most desirable from the standpoint of release of undesirable components, reactivity with liquid culture media, stability, and price Silica-alumina fibers are commerically manufactured in large quantities as refractory and insulating materials in building furnaces and as raw materials for inorganic papers. As these fibers often contain lubricants or other additives for improved processability, those which have been heated at or above temperatures where such additives can be eliminated should preferably be used.

The bulk density of the ceramic fibers of this invention ranges from 0.005 to 0.3 $g/cm^3$, preferably from

2

0.01 to 0.2 g/cm$^3$, depending upon the intended use. Fibers with a bulk density of less than 0.005 g/cm$^3$ are difficult to handle and those above 0.3 g/cm$^3$ hinder the growth of roots.

The ceramic fiber culture medium supports of this invention are hydrophilic by themselves, but they can be improved in the hydrophilic property by addition of substances harmless to the growth of plants such as nonionic surfactants and higher alcohols.

The ceramic fiber culture medium support of this invention (hereinafter referred to as the support of this invention) may be in the form of bulk, but it should preferably be in the form of a needled or unneedled blanket or loose wool in which the fibers are oriented in one direction. When the main objective is the propagation and regeneration in a culture vessel. The support of this invention can be used in the form of paper, sheet, blanket, or loose wool matching in shape to that of the culture vessel. The fibers in the support of this invention may be oriented vertically, horizontally, or at random, but the vertical orientation is desirable in consideration of the rise of liquid culture medium, the division of plantlets, and the growth of roots.

The support of this invention is used in combination with the known liquid culture media and their modifications such as Murashige-Skoog, White, Knudson, Linsmaier-Skoog, Heller, Gautheret, Nitsch-Nitsch, Ericksson, Gamborg-Miller-Ojima, and Tsukamoto-Kano. Any of these liquid culture media can be combined with the support of this invention preferably by impregnation, but also by dripping or spraying.

For culture of plant tissues with the use of the support of this invention, the support of a suitable amount is placed in a culture vessel, impregnated with a required amount of liquid culture medium, sterilized in an autoclave or the like, allowed to cool and the previously sterilized plant tissues are explanted to the culture medium thus prepared. The same care as in the case of culture with an agar culture medium is taken here, but with the use of the support of this invention, further addition of the liquid culture medium, addition of growth hormones, and cleaning and exchange of the liquid culture medium can be carried out without transplanting of the cultured tissues in an operation under sterile conditions.

The support of this invention can be used as acclimatization culture medium for plantlets, if necessary in combination with commercial liquid fertilizers. In the combined use, a liquid fertilizer prepared at a required concentration is supplied to the support of this invention by dripping, spraying, or sprinkling. For acclimatization of plantlets with the acclimatization culture medium thus prepared, when the plantlets are formed, the culture medium is washed from the root system with running tap water, and after that the plantlets are placed in a suitable amount of the acclimatization culture medium in a culture vessel with the roots of the plantlets enclosed in the medium, being kept in a greenhouse for one week to one month under suitable shielding of light and humidity. The care to be exercised here is the same as with the use of perlite or vermiculite. However, as the support of this invention is capable of holding water, sprinkling of water is usually unnecessary and the plantlets after acclimatization can be transplanted to ordinary soil culture or soilless culture without removal of the culture medium. Thus, the support of this invention provides the roots of the plantlets with a suitable amount of water and aeration and, because of high contents of voids in the support, the culture medium appears to maintain a large volume of air inside even after sprinkling and prevent a shortage of oxygen needed for the roots.

As described above, the support of this invention can be used not only as supports for culture media for the propagation, regeneration, and root formation in tissue culture but also as supports for acclimatization culture media for the acclimatization of the plantlets from the tissue culture. It is therefore possible to carry out the propagation, regeneration, and root formation of plant tissue and the acclimatization of the plantlets in succession simply by washing out the liquid culture medium previously used with water and adding the next liquid culture medium without transplanting. This consecutive procedure without transplanting markedly reduces damages to the plantlets, improves the yield of plantlets by the tissue culture method, and enhances the rate of survival of plants after acclimatization.

Moreover, the plants after acclimatization can be transplanted to normal soil culture, hydroponic culture, sand culture, gravel culture, and rock wool culture, and especially suited to rock wool culture which, similarly to the support of this invention, uses inorganic fibers.

With the use of the support of this invention, the chemical stability and physical properties of ceramic fibers make it possible for a particular liquid culture medium in combined use to manifest its full capability. Furthermore, the support of this invention has a high content of voids and most of such voids remain after the support is combined with a liquid culture medium. The voids are likely to promote the propagation, regeneration, and root formation of plant tissues or cells and the growth of cultured tissues during acclimatization. It is also likely that the liquid culture medium in combined use is present among the ceramic fibers as a result of the capillary action or adhesion and this helps the cultured tissues to absorb the liquid culture medium or facilitates removal of the liquid culture medium at the time of acclimatization.

Best mode of carrying out the invention

This invention will be illustrated with reference to the examples.

Example 1

The silica-alumina fibers ($SiO_2$, about 53 wt%; $Al_2O_3$, about 47 wt.%) used as ceramic fibers had the following properties: 1 g of the fibers released 229 ppm Al when heated with 150 ml of 0.5N hydrochloric acid at 30°C for 1 hour and showed a pH of 6.5 when immersed in an 8-fold quantity of test water of pH 7 in a

EP 0 231 387 B1

closed container at room temperature for 6 days. The fibers were oriented unidirectionally in layers, an organic lubricant was added, and the lubricated fibers were needled and then heated at about 700°C for about 20 minutes to decompose the lubricant. The resulting silica-alumina fiber culture medium support was in the form of a blanket having a bulk density of 0.13 g/cm³.

The support was cut into 2×2×2 cm cubes, 1.0 g of such cubes was placed at the bottom of a tube, 5 cm in diameter and 12 cm in height, with the fibers running vertically, 8 ml of the Murashige-Skoog culture medium (containing 0.1 mg/l) of naphthaleneacetic acid, 0.1 mg/l of benzyladenine, and 30 g/l of sucrose) was added in portions, and the tube was capped with aluminum foil, sterilized at 120°C for 15 minutes in an autoclave, and allowed to cool at room temperature.

The stem base of Cyperus pulchella (cv:nanas) soaked in a (7%) solution of sodium hypochlorite (for 10 min) in advance was cut into 2 mm cubes, explanted in the tube inside a laminar air flow cabinet, cultured at 25°C in a light and dark cycle of 12/12 hr., light intensity of 3000 lux for 3 weeks, and the rate of survival, length of shoots, total growth of shoots, number of shoots, and root formation were examined. The results are shown in Table 1.

Example 2

Silica-alumina-zirconia fibers ($SiO_2$, about 50 wt.%; $Al_2O_3$, about 35 wt.%; $ZrO_2$, about 15 wt.%) which released 77.7 ppm Al and showed a pH of 6.5 when tested as in Example 1 were converted into a silica-alumina-zirconia culture medium support in the form of a blanket (bulk density 0.13 g/cm³) in the same manner as in Example 1.

With the use of this support, the same plant tissues as in Example 1 were cultured and examined under the same conditions as in Example 1. The results are shown in Table 1.

Example 3

Silica-alumina-chromia fibers ($SiO_2$, about 55 wt.%; $Al_2O_3$, about 42 wt.% $Cr_2O_3$, about 3 wt.%) which showed a pH of 6.5 when tested as in Example 1 were converted into a silica-alumina-chromia culture medium support in the form of a blanket (bulk density 0.13 g/cm³).

With the use of this support, the same plant tissues as in Example 1 were cultured and examined as in Example 1. The results are shown in Table 1.

Comparative Example 1

Commercial purified agar for culture use (Difco Bacto agar) was added at a rate of 8 g/l to the same Murashige-Skoog culture medium as used in Examples 1—3 and the resulting agar culture medium, 8 ml, was poured into a tube of the same type as used in Examples 1—3, and the same plant tissues as in Example 1 were cultured and examined as in Example 1. The results are shown in Table 1.

Comparative Example 2

Using commercial rock wool (loose wool, hydrophilic type) for plant culture use ($SiO_2$, about 46 wt.%; $Al_2O_3$, about 13 wt.%; CaO, about 18 wt.%, MgO, about 12 wt.%; $Fe_2O_3$, about 8 wt.%), the same plant tissues as in Example 1 were cultured and examined as in Example 1. The results are shown in Table 1.

Comparative Example 3

Using commercial glass fibers in bulk ($SiO_2$, about 59 wt.%; $Al_2O_3$, about 4 wt.%, CaO, about 16 wt.%; MgO, about 5 wt.%; $B_2O_3$, about 3 wt.%; $Na_2O$, about 11 wt.%), the same plant tissues as in Example 1 were cultured and examined an in Example 1. The results are shown in Table 1.

The numerical values in Table 1 are relative to the average values of Comparative Example 1 which are taken as 100, except for the root formation which is shown in actually determined values.

As is apparent from Table 1, the use of ceramic fiber culture medium supports in Examples 1—3 has considerably improved the survival rate, growth of shoots, and root formation compared with Comparative Examples 1—3.

4

# EP 0 231 387 B1

TABLE 1

| | Example | | | Comparative Example | | |
|---|---|---|---|---|---|---|
| | 1 | 2 | 3 | 1 | 2 | 3 |
| Survival rate | 161 | 169 | 154 | 100 | 146 | 38 |
| Length of shoots | 135 | 147 | 138 | 100 | 71 | 3 |
| Total growth of shoots | 134 | 131 | 138 | 100 | 80 | 2 |
| No. of shoots | 72 | 76 | 84 | 100 | 80 | 8 |
| Root formation (%) | 95 | 91 | 95 | 0 | 47 | 0 |

Example 4

The same silica-alumina fibres as in Example 1 were converted into a support in the form of a blanket (bulk density 0.1 g/cm$^3$).

The support was cut into 2×2×2 cm cubes 0.8 g of such cubes were placed at the bottom of a tube, 5 cm in diameter and 12 cm in height, with the fibers running vertically, and using 8 ml of the same Murashige-Skoog culture medium as in Example 1, the 2 mm cubes of the stem base of Cyperus pulchella (cv:nanas) were cultured for 3 weeks under the same conditions as in Example 1, and the survival rate, length of shoots, number of shoots, and root formation were examined. The results are shown in Table 2.

As in Examples 1—3, the control culture was carried out here as in Comparative Example 1 and the results were expressed relative to the average values, taken as 100, of the control.

TABLE 2

| | |
|---|---|
| Survival rate | 117 |
| Length of shoots | 237 |
| No. of shoots | 106 |
| Root formation | 139 |

As is apparent from Table 2, the use of the silica-alumina culture medium support here has considerably promoted the growth of shoots.

The plantlets of Cyperus obtained in this Example were taken out of the tube together with the silica-alumina fiber culture medium support, washed with running water, placed with the support in a plastic container, subjected to acclimatization in a greenhouse with 75% shielding of light for 10 days, and the rate of survival was examined.

Separately, a culture soil for acclimatization use consisting of a mixture of 2 weight parts peat moss, 1 weight part perlite, and 0.5 weight part vermiculite was placed in a plastic container of the same type as in this Example to a thickness of 2 cm. The plantlets of Cyperus obtained in the control culture in this Example (the plantlets were removed from the tubes and the agar was washed from the root system with running tap water) were planted, acclimatized, and examined under the same conditions.

With the rate of survival of the control taken as 100, that of this example is 143. Thus, the use of the silica-alumina fiber culture medium support improved the rate of survival at the time of acclimatization.

Example 5

Using the same silica-alumina fiber culture medium support as in Example 4, 5 mm sections of the tip of the runner of Nephrolepis exaltata var. marshallii were explanted on the culture medium as in Example 4, cultured for 8 weeks under the same conditions as in Example 1, and survival rate, length of shoots, number of shoots, and root formation were examined. The results are shown in Table 3.

Example 6

The same silica-alumina-zirconia fibers as in Example 2 were converted into a silica-alumina-zirconia fiber culture medium support in the form of a blanket (bulk density 0.1 g/cm$^3$). Using this support, the same plant tissues as in Example 5 were cultured and examined as in Example 5. The results are shown in Table 3.

## EP 0 231 387 B1

As in Examples 1—3, the results of Examples 5 and 6 were expressed relative to the control, taken as 100, cultured as in Comparative Example 1.

As is apparent from Table 3, the culture medium supports prepared from two different ceramic fibers were found to promote the growth of shoots and root formation considerably compared with the control.

TABLE 3

|  | Example | | Control |
|---|---|---|---|
|  | 5 | 6 | Agar culture medium |
| Survival rate | 100 | 100 | 100 |
| Length of shoots | 135 | 152 | 100 |
| No. of shoots | 145 | 156 | 100 |
| Root formation (%) | 33 | 52 | 0 |

Example 7

The same silica-alumina fiber culture medium support (bulk density 0.1 g/cm$^3$) as in Example 4 was cut into 1.8 cm cubes, about 0.6 g of such cubes was placed in a bottle, and 6 ml of the same Murashige-Skoog culture medium as in Example 1 was poured into the tube. Sections of a leaf of Saintpaulia ionantha Hybrida A, in 2 mm cubes, were explanted on the culture medium as in Example 1, cultured for 40 days under the same conditions as in Example 1, and the survival rate, diameter of callus, length of shoots, number of shoots, and root formation were examined. Expressed relative to the averages, taken as 100, of the control cultured in an agar culture medium as in Comparative Example 1, the results were: survival rate 144, diameter of callus 269, length of shoots 288, number of shoots 260, and root formation 135.

As is apparent from the results, the use of the silica-alumina fiber support in this Example considerably promoted the growth of callus and regeneration and growth of shoots.

Example 8

The same silica-alumina fibers as in Example 1 were converted into a silica-alumina fiber culture medium support in the form of a blanket (bulk density 0.07 g/cm$^3$).

The support was cut into 2×2×2 cm cubes, 0.56 g of such cubes was placed on the bottom of a tube, 5 cm in diameter and 12 cm in height, with the fibers running vertically, sections of a leaf of Saintpaulia ionantha Hybrida B, in 2 mm cubes, were explanted on the culture medium as in Example 1, cultured for 13 weeks under the same conditions as in Example 1, and the survival rate, callus formation, length of shoots, number of shoots, and root formation were examined. The results expressed relative to the averages, taken as 100, of the control cultured in an agar culture medium as in Comparative Example 1 were: survival rate 100, callus formation 100, length of shoots 276, number of shoots 105, and root formation 100. As is apparent from these results, the use of the silica-alumina fiber culture medium support of this Example considerably promoted the growth of shoots.

Example 9

The silica-alumina fiber culture medium support prepared in Example 1 was cut into 2 cm cubes, a cut was made in the center of such cubes, and the acclimatization culture medium was prepared from such cut cubes.

The plantlets of Cyperus pulchella (cv:nanas) obtained by tissue culture (in the Murashige-Skoog culture medium for 20 days) were removed from the tubes and the agar was washed from the root system with running tap water. Then the plantlets were inserted into the cut in the aforesaid acclimatization culture medium.

The plantlets thus planted were placed in a plastic culture vessel, acclimatized in a greenhouse with 75% shielding of light for 10 days, and the rate of survival was examined. Water was sprinked once a day until the culture medium was saturated with water and any surplus water was discharged from the culture vessel.

As a control in this Example, a culture soil composed of 2 weight parts peat moss, 1 weight part perlite, and 0.5 weight part vermiculite was introduced as acclimatization culture medium into a plastic culture vessel of the same type as in Example 4 to a thickness of 2 cm, the same plantlets as used in Example 4 were placed in the acclimatization culture medium and acclimatized under the same conditions as in this Example above. The rate of survival was 143 with reference to that of the control as 100.

Example 10

The plantlets of Alocasia macrorhiza cultured in the Murashige-Skoog culture medium in a tube for 60

6

days were planted in 3×3×3 cm cubes of acclimatization culture medium and acclimatized under the same conditions as in Example 9, and the rate of survival was examined.

The control in this Example was prepared and acclimatized under the same conditions as in Example 9 except for the thickness of the soil culture being 3 cm. The rate of survival of this Example was 125 relative to that of the control as 100.

The plants acclimatized in this Example and its control were planted and their growth was examined. The test plant and the acclimatization culture medium were inserted into a hole, 3 cm in diameter and 3 cm in depth, made at the center of a 7×7×6 cm commercial rock wool block for plant culture use, the block was placed in a greenhouse with 45% shielding of light, and cultured for 30 days while sprinkled once a day with a 2000-fold dilution of a Hyponex stock solution (liquid fertilizer manufactured by Murakami Bussan K.K.; 5 (N)-10 (P)-5 (K) and micronutrients).

As a control, the plant acclimatized as the control in this Example was taken out of the culture vessel together with as much of the culture soil as possible, and planted in the center of a rock wool block for plant culture using the soil culture used in the acclimatization and cultviated in a greenhouse as above.

When the growth of the plants was compared in terms of the number of leaves, the test plant in this Example produced growth of 150 relative to 100 for the control.

Industrial applicability

As described above, a culture medium support of this invention is used in combination with a liquid culture medium for plant tissue culture, and if necessary in combination with commercial liquid fertilizers as supports for acclimatization culture medium for plantlets.

**Claims**

1. A culture medium support for plant tissue culture comprising ceramic fibers releasing substantially no components harmful to plant tissue culture and used in combination with a liquid culture medium, wherein the ceramic fibers are silica-alumina fibers.

2. A culture medium support for plant tissue culture according to Claim 1 in which the ceramic fibers do not substantially react with the liquid culture medium.

3. A culture medium support for plant tissue culture according to Claim 1 in which the ceramic fibers are hydrophilic.

4. A culture medium support for plant tissue culture according to Claim 1, in which the ceramic fibers are one or more kinds of ceramic fibers selected from silica-alumina fibers, silica-alumina-zirconia fibers and silica-alumina-chromia fibers.

5. A culture medium support for plant tissue culture according to Claim 1 in which the ceramic fibers are oriented vertically.

6. A culture medium support for plant tissue culture according to Claim 1 in which the bulk density of the support ranges from 0.005 to 0.3 g/cm³.

**Patentansprüche**

1. Träger für Kulturmedium für Pflanzengewebekulturen aus Keramikfasern, der praktisch keine für die Pflanzengewebekulturen schädliche Bestandteile freisetzt und in Kombination mit einem flüssigen Kulturmedium verwendet wird, in dem die Keramikfasern Siliciumdioxid-Aluminiumoxid-Fasern sind.

2. Träger für Kulturmedium für Pflanzengewebekulturen nach Anspruch 1, in dem die Keramikfasern praktisch nicht mit dem flüssigen Kulturmedium reagieren.

3. Träger für Kulturmedium für Pflanzengewebekulturen nach Anspruch 1, in dem die Keramikfasern hydrophil sind.

4. Träger für Kulturmedium für Pflanzengewebekulturen nach Anspruch 1, in dem die Keramikfasern eine oder mehrere Arten von Keramikfasern sind, ausgewählt unter Siliciumdioxid-Aluminiumoxid-Fasern, Siliciumdioxid-Aluminiumoxid-Zirkoniumdioxid-Fasern und Siliciumdioxid-Aluminiumoxid-Chromoxid-Fasern.

5. Träger für Kulturmedium für Pflanzengewebekulturen nach Anspruch 1, in dem die Keramikfasern senkrecht orientiert sind.

6. Träger für Kulturmedium für Pflanzengewebekulturen nach Anspruch 1, in dem die Schüttdichte des Trägers von 0,005 bis 0,3 g/cm³ beträgt.

**Revendications**

1. Support de milieu de culture pour la culture de tissus végétaux, comprenant des fibres céramiques ne libérant sensiblement pas de composants nocifs pour la culture de tissus végétaux et utilisé en combinaison avec un milieu de culture liquide, dans lequel les fibres céramiques sont des fibres de silice-alumine.

2. Support de milieu de culture pour la culture de tissus végétaux selon la revendication 1, dans lequel les fibres céramiques ne réagissent sensiblement pas avec le milieu de culture liquide.

7

3. Support de milieu de culture pour la culture de tissus végétaux selon la revendication 1, dans lequel les fibres céramiques sont hydrophiles.

4. Support de milieu de culture pour la culture de tissus végétaux selon la revendication 1, dans lequel les fibres céramiques sont constituées par une ou plusieurs sortes de fibres céramiques choisies parmi les fibres de silice-alumine, les fibres de silice-alumine-zircone et les fibres de silice-alumine-oxyde de chrome.

5. Support de milieu de culture pour la culture de tissus végétaux selon la revendication 1, dans lequel les fibres céramiques sont orientées verticalement.

6. Support de milieu de culture pour la culture de tissus végétaux selon la revendication 1, dans lequel la masse volumique apparente du support se situe dans la plage de 0,005 à 0,3 g/cm$^3$.